# EUROPEAN PATENT SPECIFICATION

(11) **EP 0 736 002 B1**
(45) Date of publication and mention of the grant of the patent: **03.04.2002**
(21) Application number: 95902010.8
(22) Date of filing: 19.12.1994
(51) Int. Cl.: C07C 257/14, C12N 15/87, A61K 47/48, A61K 9/127, C12N 5/10, A01K 67/027, A01H 5/00, C07C 257/06, A61K 7/00

(54) **COMPOUND CAPABLE OF INTRODUCING AT LEAST ONE MOLECULE INTO A CELL**
VERBINDUNG GEEIGNET ZUR EINFÜHRUNG VON WENIGSTENS EINEM MOLEKÜL IN EINE ZELLE
COMPOSE POUVANT INTRODUIRE AU MOINS UNE MOLECULE DANS UNE CELLULE

(30) Priority: 20.12.1993 US 170124
(43) Date of publication of application: 09.10.1996
(73) Proprietor: Biotech Tools S.A., 1120 Bruxelles (BE)
(72) Inventor: RUYSSCHAERT, Jean-Marie, B-1640 Brussels (BE); FUKS, Robert, B-1180 Brussels (BE)
(74) Representative: Van Malderen, Michel
(86) International application number: BE9400096
(87) International publication number: WO9517378

(56) References cited:
- WO-A-91/15501
- JOURNAL OF THE CHEMICAL SOCIETY. CHEMICAL COMMUNICATIONS, no.13, 1 July 1986 pages 1060 - 1062 FABIENNE DEFRISE-QUERTAIN ET AL cited in the application
- BIOCHEMICAL AND BIOPHYSICAL RESEARCH COMMUNICATIONS, vol.203, no.3, 30 September 1994 pages 1622 - 1628 JEAN-MARIE RUYSSCHAERT ET AL
- THE JOURNAL OF ORGANIC CHEMISTRY, vol.54, no.18, 1 September 1989 pages 4335 - 4344 ARNOLD J. GUTIERREZ ET AL
- TETRAHEDRON LETTERS, vol.27, no.40, 1986 pages 4837 - 4840 K.J. SHEA ET AL
- CHEMICAL ABSTRACTS, vol. 95, no. 17, 26 October 1981, Columbus, Ohio, US; abstract no. 149705c, page 580 ;column L ; & ARM. KHIM. ZH., vol.34, no.5, 1981, MOSCOW pages 370 - 374
- Gene Therapy (1995) 2, 710-722 BIOCHEMICAL AND BIOPHYSICAL RESEARCH COMMUNICATION, Vol. 203, No. 3, 1994 FEBS Letters 414, (1997), 187-192 VIROLOGY 189, 775-777 (1992)

## Description

### Object of the invention

The invention relates to a vector ; to the cell, transformed by said vector.

The invention also relates to the process for the synthesis of said compound and to the process for the production of said vector.

Another aspect of the invention relates to the use of the compound and/or vector for the introduction in vitro of at least one molecule into a cell.

### Technological background and prior state of the art which form the basis of the invention

Various processes are used in genetic engineering and/or in pharmacy to introduce molecules such as nucleic acids or active therapeutic agents into cells.

Transfection is a method which is widely used for introducing genetic material into cells, for studying the expression of genes, and for developing strategies for gene therapy.

Numerous experimental procedures using physical modifications (microinjection, electroporation and the like) or chemical modifications (dextran phosphate, calcium phosphate and the like) of membranes have been developed with varying success for introducing nucleic acids into cells using this method.

Another axis of research relates to the development of new amphiphilic cationic vectors which have demonstrated their effectiveness and their ease of use for causing the genetic material to penetrate into cells in vitro (P.L. Felgner et al., Proc. Natl. Acad. Sci., USA, 84, pp. 7413-7417 (1987)).

Some of these vectors such as the Lipofectin® N-(2,3-dioleyloxy)propyl-N,N,N-trimethylammonium chloride (DOTMA) from GIBCO BRL or the Transfection-reagent® N-(2,3-dioleoyloxy)propyl-N,N,N-trimethylammonium methyl sulfate (DOTAP) from Boehringer Mannheim GmbH, have been commercialized.

Patent Application WO91/15501 (YALE UNIVERSITY) also describes a positively charged reagent consisting of a neutral phospholipid such as dioleoylphosphatidylethanolamine and a cationic lipid such as stearylamine, a tertiary amine or a benzothorium salt for the transfection of nucleic acids.

Nevertheless, these products require large quantities of reagents in order to obtain an effective transfection of cells and therefore increase the cytotoxicity of the products and their cost (thus, the price of the "Lipofectin® " product (DOTMA) is US 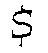 145/ml, equivalent to US 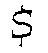 10 per transfection).

In addition, Lipofectin® (DOTMA) has the additional disadvantage of not being capable of being added to a serum.

The document "Vesicle Formation by double long-chain Amidines" (Fabienne Defrise-Quertain et al.) (J. Chem. Soc., Chem. Commun., 1986, p. 1050 to 1062) describes the formation of vesicles consisting of 3-tetradecylamino-N-tert-butyl-N'-tetradecylopropionamidine.

### Aims of the invention

The aim of the present invention is to produce a a new vector capable of introducing at least one molecule into a cell, without exhibiting the disadvantages of the prior state of the art.

The invention is also intended for producing a pharmaceutical or cosmetic composition comprising said vector and/or said cell transformed by said vector.

An additional aim of the present invention is intended for producing a vector which can be used in a serum.

### Characteristic elements of the invention

The present invention relates to a vector consisting of a positively charged vesicle comprising a least one compound of general formula: 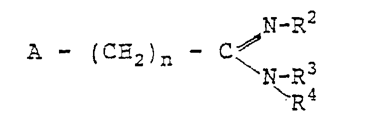 in which
- A is chosen from the group consisting of the radicals comprising a hydrophilic group or a CH₂ group and a hydrophobic group;
- n is a positive integer, preferably n>1; and
- R², R³ and R⁴ are chosen from the group consisting of a hydrogen atom, saturated alkyl chains, unsaturated alkyl chains and substituted alkyl chains.

Preferably, A represents a radical of formula in which
- R¹ is chosen from the group consisting of a hydrogen atom, saturated alkyl chains, unsaturated alkyl chains and substituted alkyl chains, and
- X represents a divalent atom or a divalent group of atoms chosen from the group consisting of
- the carbon and/or oxygen derivatives: 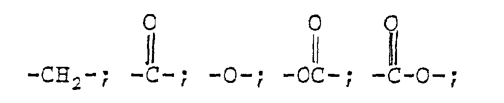
- the sulfur derivatives: 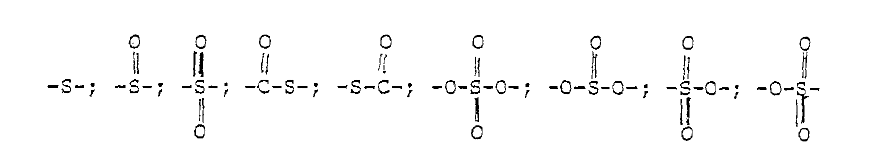 and their selenium and tellurium analogs;
- the nitrogen derivatives;

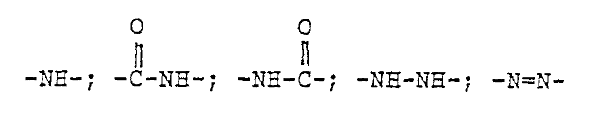

Advantageously, R¹ and R³ or R¹ and R⁴ are chosen from the group consisting of hydrocarbon chains containing 12 or more carbon atoms, preferably 12 to 18 carbon atoms.

According to a preferred embodiment of the invention, the compound corresponds to the following formula: 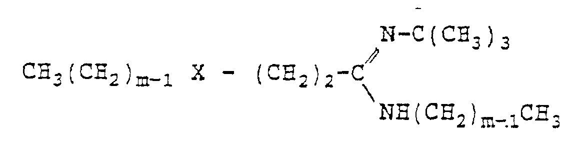 in which 12 ≤ m ≤ to 18.

The vector according to the invention consist of vesicle having a membrane comprising the compound associated with at least one molecule chosen from the group consisting of nucleic acids such as plasmids, messenger RNAs, antisense RNAs, cDNAs, synthetic oligonucleotides and the like which are capable of genetically transforming a cell, polypeptides or optionally glycosylated proteins.

An aspect of the invention relates to a process for the introduction of at least one molecule chosen from the group consisting of nucleic acids, polypeptides or optionally glycosylated proteins, into a cell, in which process said cell is brought into contact with said molecule and a compound and/or a vesicle according to the invention. Likewise, this introduction process can be achieved by bringing said cell into contact with a vector according to the invention.

According to a first embodiment of the process of the invention, a cell is treated in vitro in order to produce plants or animals which are transgenic or in order to produce gene therapy, in particular for the treatment of cellular disorders such as cancer or infections such as viral or bacterial infections or the like.

According to the second preferred embodiment of the process of the invention, a cell is treated in vitro in order to produce recombinant microorganisms, plants and/or animals which are transgenic or in order to produce gene therapy.

The present invention also relates to the cell transformed by the vector according to the invention, as well as a cosmetic or pharmaceutical composition such as a vaccine, comprising the vector according to the invention and/or the transformed cell according to the invention.

The invention also relates to a process for the production of the vector according to the invention in which at least one molecule chosen from the group consisting of nucleic acids, polypeptides and optionally glycosylated proteins are reacted with a vesicle according to the invention.

A final aspect of the invention relates to the use of the vector and/or the compound according to the invention for the introduction of one or more molecules into a cell in vitro

### Description of a preferred embodiment of the invention

The present invention is based on the unexpected fact that a compound of general formula: 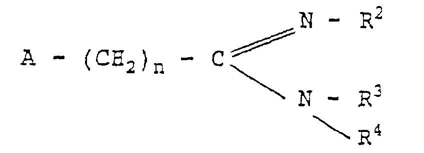 in which
- A is chosen from the group consisting of the radicals comprising a hydrophilic group or a CH₂ group and a hydrophobic group;
- n is an integer, preferably > 1,
- R², R³ and R⁴ are chosen from the group consisting of a hydrogen atom, saturated alkyl chains, unsaturated alkyl chains and substituted alkyl chains,
can form vesicles and combine with other molecules to form a vector capable of introducing said molecules into an animal or plant cell or a microorganism.

By "other molecules" there is understood any molecular structure such as a nucleic acid, a polypeptide or an optionally glycosylated protein, capable of modifying the state of said cell, in particular the physicochemical state or the genome of said cell.

### 1) Synthesis of 3-alkylamino-N-tert-butyl-N'-alkylpropionamidine (8)

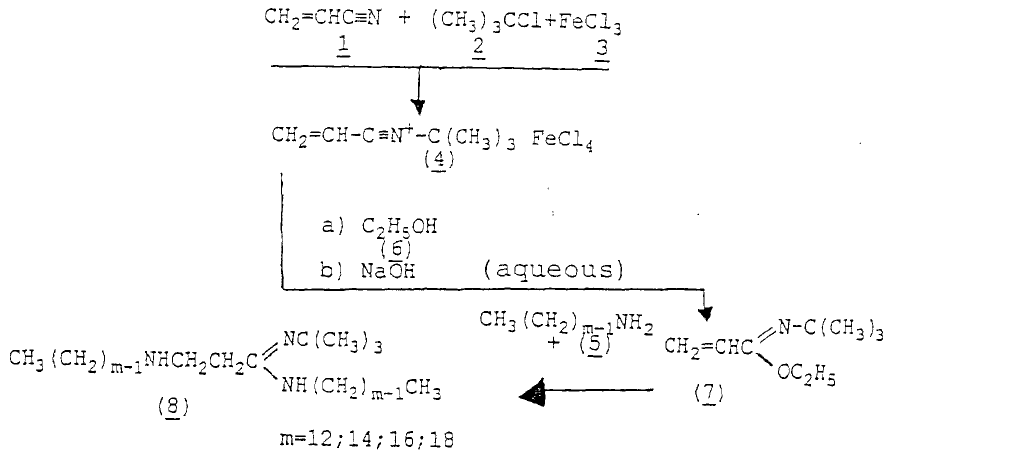

Acrylonitrile is converted to a nitrilium salt 4, which is converted "in situ" by ethanol 5 to ethyl N-tert-butylpropenimidate 7, a colorless liquid which is stable at cold temperature (refrigerator) for several weeks. Nevertheless, left at room temperature, compound 7 becomes slowly degraded by polymerization. The imidate 7 (1 equivalent), treated with the alkylamine 5 (2 equivalents) at 65°C, gives in all cases the double-long-chain amidine 8 with yields of 60-65% (see Table 1).
To a stirred suspension of 65 g (0.4 mol) of anhydrous ferric chloride in 300 ml of anhydrous dichloromethane at -25°C, are added 28 ml (0.42 mol) of acrylonitrile 1. After 5 minutes, the red suspension is cooled to -35°C and 46 ml (0.42 mol) of tert-butyl chloride (2) are then added. The color passes from red to ochre thus forming N-tert-butylacrylonitrilium tetrachloroferrate (4). The suspension is cooled to -80°C and 46.8 g (10.8 mol) of absolute ethanol (dried and distilled over magnesium) are added thereto, with stirring. The temperature of the mixture is allowed to rise up to -20°C and then it is added to 800 ml of 1.5 M aqueous sodium hydroxide cooled to 0°C. The mixture is then separated after settling has taken place at room temperature. The aqueous phase is extracted 3 times with 100 ml of dichloromethane. The combined organic phases are dried over magnesium sulfate and then filtered. After addition of a spatula-tip quantity of hydroquinone, the solution is evaporated using a rotary evaporator, the bath should not exceed 30°C. The liquid residue is then distilled under vacuum on a small column provided with a dephlegmator at 56°C under 30 torr. Yield 41.01 g, equivalent to 66% of 7. A suspension of 2 equivalents of alkylamine 5 in 1 equivalent of acrylimidate 7 is stirred under vacuum produced by a water-jet pump and heated to 65°C. After 6 h, the mixture is allowed to cool. It is taken up in hexane and filtered on a γ-Tonerde alumina column. The filtrate is evaporated under vacuum and then pump-dried, with stirring, and by melting and solidifying the amidine 8 several times (see Table 1).
The physical, spectroscopic and analytical characteristics are given in Table 2.

**Table 1**

| Synthesis of 3-alkylamino-N-tert-butyl-N'-alkylpropionamidine 8 from ethyl N-tert-butylacrylimidate 7. | | | |
|---|---|---|---|
| Alkylamine 5 | Acrylimidate 7 | Hexane and Al₂O₃ | Yield |
| m = 12; 18.5 g (0.1 mol) | 7.75 g (0.05 mol) | 50 ml; 200 g | 15.47 g; 65% |
| m - 14; 21.3 g (0.1 mol) | 7.75 g (0.05 mol) | 50 ml; 200 g | 17.82 g; 66% |
| m = 16; 19.28 g (0.08 mol) | 6.2 g (0.04 mol) | 60 ml; 160 g | 14.6 g; 62% |
| m = 18; 21.5 g (0.08 mol) | 6.2 g (0.04 mol) | 80 ml; 160 g | 15.69 g; 61% |

**Table 2**

| Physical, Analytical and Spectroscopic data of amidines (8) | | | | | |
|---|---|---|---|---|---|
| amidine(8) | m.p. °C (a) | Molecular formulae (M) | Elementary analysis (b) | | Mass Spectra (c) M+ |
| | | | calculated (%) | found (%) | |
| m=12 | 27-29 | C₃₁H₆₅N₃ | C 77.59 | 77.52 | 479 |
| | | (479.85) | H 13.65 | 13.90 | |
| | | | N 8.76 | 8.72 | |
| m=14 | 31-33 | C₃₅H₇₃N₃ | C 78.43 | 78.13 | 535 |
| | | (535.96) | H 13.73 | 14.10 | |
| | | | N 7.84 | 7.72 | |
| m=16 | 37-39 | C₃₉H₈₁N₃ | C 79.11 | 79.43 | 591 |
| | | (592.06) | H 13.79 | 14.22 | |
| | | | N 7.10 | 7.06 | |
| m=18 | 40-42 | C₄₃H₈₉N₃ | C 79.68 | 79.62 | 647 |
| | | (648.17) | H 13.84 | 14.05 | |
| | | | N 6.48 | 6.57 | |

| | | | | | |
|---|---|---|---|---|---|
| (a) Determined on a Leitz Mikroskopheiztisch 350 and the m.ps. are not corrected | | | | | |
| (b) Elementary analyses carried out at "Searl-Continental Pharma Laboratory" | | | | | |
| (c) Determined on a Mass Spectrometer Hitachi RMU-6D | | | | | |

### 2) Formation of vesicles consisting of 3-tetradecylamino-N-tert-butvl-N'-tetradecylpropionamidine (pure diC₁₄-amidine (1 mg/ml))

A solution of di C₁₄-amidine is prepared in ethanol (100 mg/ml). 30 µl (3 mg) of this solution are rapidly injected, by means of a Hamilton syringe, into 3 ml of Tris/HCl buffer (0.01 M, pH 7.4), heated to 30°C. During the operation, the solution is gently stirred by means of a magnetic bar. The vesicles form immediately.

### 3) Formation of vesicles consisting of 3-tetradecylamino-N-tert-butyl-N'-tetradecylpropionamidine ((8), m=14) and phosphatidylethanolamine (diC₁₄-amidine/PE (1 mg/ml))

1.25 mg of diC₁₄-amidine and 1.75 mg of PE (bovine brain) dissolved in chloroform (10 mg/ml) are dried under a nitrogen stream so as to form a thin film on the walls of the tube; traces of the solvent are removed under vacuum overnight. The film is then mechanically stirred in the presence of 3 ml of Tris/HCl buffer (0.01 M, pH 7.4). The cloudy suspension obtained is then sonicated (Branson B-12 Sonifier, 60 W) under a nitrogen stream and on an ice bath until a clear suspension is produced (about 10 min).

Various in vitro transfection methods are given in the following examples given solely by way of illustration of the invention.

### Example I

### 1) Formation of the vector vesicles-plasmid DNA

5 µg of plasmid DNA and 11 µg (11 µl) of pure diC₁₄-amidine suspension (or 25 µg (25 µl) of diC₁₄-amidine/PE suspension) are separately diluted in a final volume of 50 µl with distilled water and sterilized water Both solutions are then mixed and incubated for 15 min at room temperature.

### 2) Transfection of adherent cells

The cells are cultured in 6-well culture dishes until they are 80-90% confluent.

The suspension containing the complex (diC₁₄-amidine-DNA or diC₁₄-amidine/PE-DNA) is gently mixed with 2 ml of culture medium. The culture medium used for the transfection operation is identical to the usual culture medium for the cells to be transformed. The optional presence of serum in the medium has no inhibitory action on the transfection process and can even prove necessary for many cell lines (especially in suspension). The mixture thus formed constitutes the "transfection medium". The cells (about 10⁶) are washed with the culture medium and the transfection medium is added. The medium is incubated for the desired period. This period may depend on the cell line to be transfected but an incubation time of 2-3 h is generally used. After incubation, the transfection medium is replaced with fresh medium; 6 hours later, the cells can be transferred into 100 mm culture dishes.

### 3) Transfection of cells in suspension

The suspension containing the complex (diC₁₄-amidine-DNA or diC₁₄-amidine/PE-DNA) is gently mixed with 10 ml of medium. The mixture thus formed constitutes the "transfection medium". Aliquots of 10⁶ cells are centrifuged and the pellets are resuspended in the transfection medium. The cell suspensions are distributed into 100 mm culture dishes. The suspensions are incubated for the desired period; after incubation, the cells are centrifuged and resuspended in 10 ml of fresh medium and then cultured normally.

CHO (Chinese Hamster Ovary) cells are cultured in F12 medium supplemented with 1% glutamine and 1% penicillin/streptomycin and containing 10% FCS (Fetal Calf Serum. K562 (human myeloid) cells are cultured in RFMI medium supplemented with antibiotics and non-essential amino acids and containing 10 % FCS (0.5-1 × 10⁶ cells/ml). Both cell lines are cultured under a CO₂ atmosphere (37°C, 5% CO₂).

The plasmid used is derived from the commercial plasmid pCMV5 into which the bacterial replication origin S1, the ampicillin resistance gene and the CAT gene of bacterial origin have been inserted. It is approximately 5000 base pairs in size and is stored at a concentration of 1 mg/ml.

The general procedure described above was followed; 5 µg (5 µl) of plasmid DNA and 11 µg (11 µl) of pure diC₁₄-amidine suspension (or 25 µg (25 µl) of diC₁₄-amidine/PE suspension) prepared as described above are used. The CHO cells (adherent) are plated in the 6-well culture dishes the day before the experiment in an amount of 10⁶ cells/well; while the K562 cells (in suspension) are used in an amount of 10⁶ cells per experiment.

The incubation was carried out for 3 h for the two cell lines. The transfection medium is the normal culture medium (with serum) with which vesicle vector (pure diC₁₄-amidine or diC₁₄-amidine/PE/DNA) has been mixed under the conditions described in the general procedure.

After incubation, the cells are treated as described above; the CHO cells are transferred after 6 h into 100 mm culture dishes. The two cell lines continue to grow for 36 h before the CAT assay performed as described by Gorman et al., B.H. 1982), mol. Cell. Biol. 2, 1044-1051; in brief, the cell lysates are incubated at 37°C with [¹⁴C]chloramphenlcol and acetyl CoA for 2 h and extracted with ethyl acetate. The organic phase is eluted by TLC (thin-layer chromatography) with chloroform/ methanol 95:5. The acetylation of chloramphenicol is determined by autoradiography on the chromatograms and counting of the spots on the plate.

### Example II

Table 3 below gives the comparison of the transfection efficiency of the CAT gene for diC₁₄ and the product DOTAP® marketed by Boehringer Mannheim, expressed as percentage of acetylated chloramphenicol in the cellular extract, under identical experimental conditions.

The cell lines of this example are CHO cells (adherent cells) and K562 cells (suspension cells).

The composiolon of the vector comprises: µg quantities of plasmid DNA and µg quantities of cationic vesicles.

**Table 3**

| | Adherent cells | | Suspension cells | |
|---|---|---|---|---|
| Composition of vector | di C₁₄ | DOTAP | diC₁₄ | DOTAP |
| 2 + 5 (µg) | 63% | 32% | 60% | 29% |
| 5 + 10 (µg) | 65% | 40% | 68% | 36% |

The diC₁₄ allows transfection efficiencies with quantities two to three times lower than the available commercial products, namely DOTAP® from Boehringer Mannheim and Lipofectin® from Gibco BRL, in addition, the transfection efficiency advantageously limits the cytotoxicity of the product used.

### Example III

### Transfection of genetic material

100 µg of BLV (Bovine Leukemia Virus) genome and 200 µg of amidine diC14 were hypodermically injected into a sheep. After 1 month, the virus envelope protein (51 kD) is detected immunochemically in the sheep's serum.

## Claims

1. A vector consisting of a positively charged vesicle comprising at least one compound of formula: 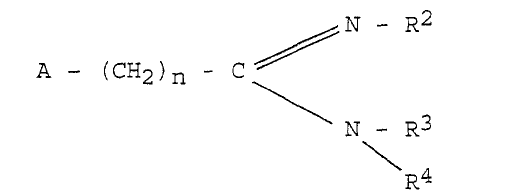 in which:
- A is selected from the group consisting of the radicals comprising a hydrophilic group or a CH₂ group and a hydrophobic group;
- n is a positive integer; and
- R², R³ and R⁴ are selected from the group consisting of a hydrogen atom, saturated alkyl chains, unsaturated alkyl chains and substituted alkyl chains,
said vesicle being combined with at least one molecule selected from the group consisting of nucleic acids, polypeptides, glycosylated polypeptides, proteins and glycosylated proteins.

2. The vector as claimed in claim 1, wherein the vesicle comprises at least one compound of formula: 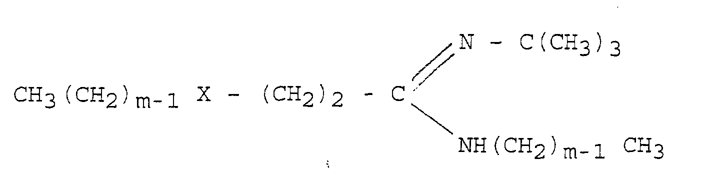 in which:
- m is an integer from 12 to 18, and
- X is chosen from the group consisting of 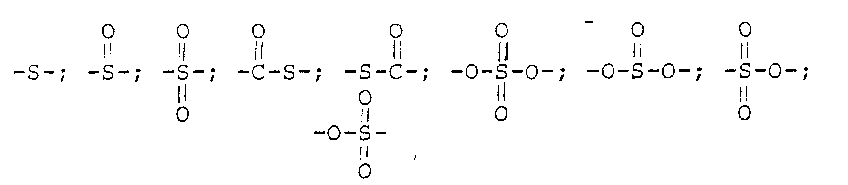 their selenium analogs, their tellurium analogs,

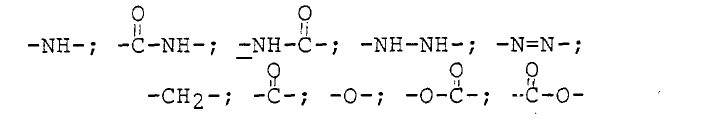

3. A process for the introduction of at least one molecule selected from the group consisting of nucleic acids, polypeptides, glycosylated polypeptides, proteins and glycosylated proteins, in vitro into a cell, in which said cell is brought in contact with said molecule and a compound of formula: 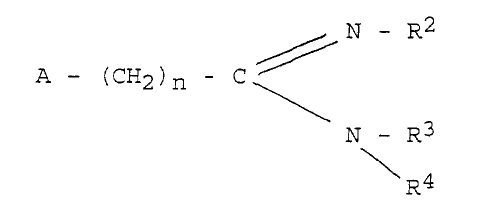 in which:
- A is selected from the group consisting of the radicals comprising a hydrophilic group or a CH₂ group and a hydrophobic group;
- n is a positive integer; and
- R², R³ and R⁴ are selected from the group consisting of a hydrogen atom, saturated alkyl chains, unsaturated alkyl chains and substituted alkyl chains.

4. The process as claimed in claim 3, in which said cell is brought into contact with said molecule and a compound of formula: 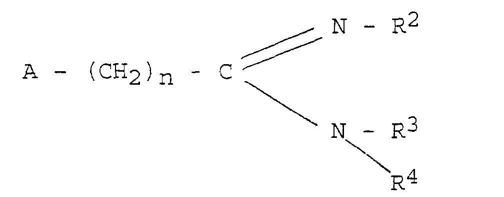 in which:
- A represents a radical of formula -X-R¹, with R¹ is selected from the group consisting of a hydrogen atom, saturated alkyl chains, unsaturated alkyl chains and substituted alkyl chains, and with X selected from the group consisting of: 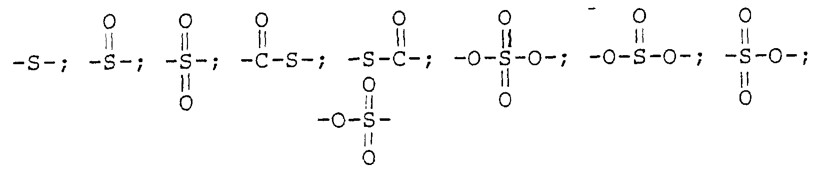 their selenium analogs, their tellurium analogs,

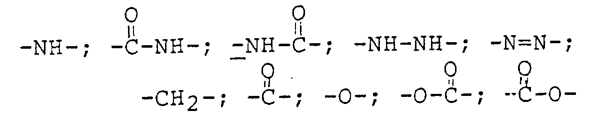

5. The process according to the claim 3, in which said cell is brought into contact with said molecule and with a positively charged vesicle whose membrane comprises at least one compound of formula:

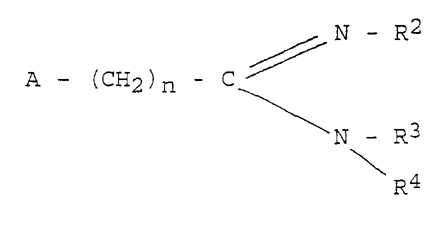

6. The process as claimed in claim 5, in which the vesicle comprises at least one compound of formula: 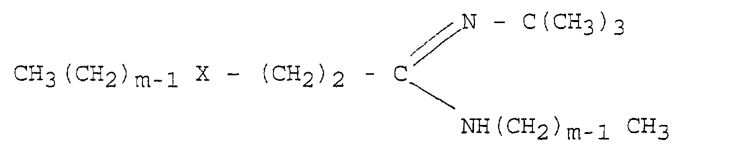 in which m is an integer from 12 to 18.

7. The process as claimed in claim 3, in which said cell in brought into contact with a vector consisting of a positively charged vesicle whose membrane comprises at least one molecule of formula: 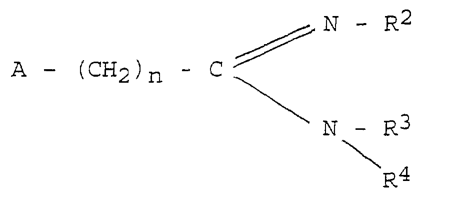 said vesicle being combined with a molecule selected from the group consisting of nucleic acids, polypeptides, glycosylated polypeptides, proteins and glycosylated proteins.

8. A cell transformed by a process in which said cell is brought into contact with a molecule selected from the group consisting of nucleic acids, polypeptides, glycosylated polypeptides, proteins and glycosylated proteins and with a compound of formula: 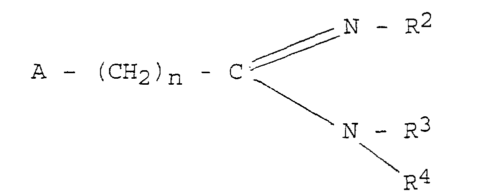 in which:
- A is selected from the group consisting of the radicals comprising a hydrophilic group or a CH₂ group and a hydrophobic group;
- n is a positive integer; and
- R², R³ and R⁴ are selected from the group consisting of a hydrogen atom, saturated alkyl chains, unsaturated alkyl chains and substituted alkyl chains.

9. The cell as claimed in claim 8, said cell being transformed by bringing it into contact with a vector consisting of a positively charged vesicle whose membrane comprises at least one molecule of formula: 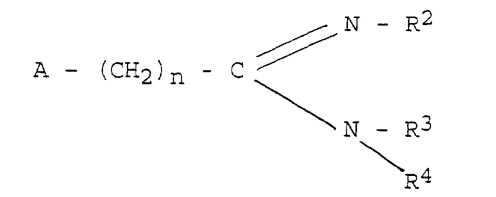 said vesicle being combined with a molecule selected from the group consisting of nucleic acids, polypeptides, glycosylated polypeptides, proteins and glycosylated proteins.

10. A pharmaceutical composition comprising a suitable pharmaceutical carrier and a product chosen from the group consisting of:
- the vectors consisting of a positively charged vesicle comprising at least one compound of formula: 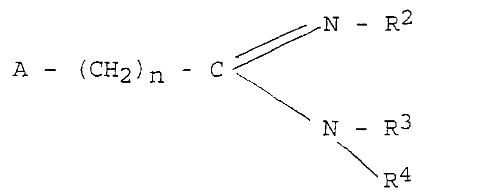 in which:
- A is selected from the group consisting of the radicals comprising a hydrophilic group or a CH₂ group and a hydrophobic group;
- n is a positive integer; and
- R², R³ and R⁴ are selected from the group consisting of a hydrogen atom, saturated alkyl chains, unsaturated alkyl chains and substituted alkyl chains,
said vesicle being combined with at least one nucleic acid molecule,
and
- the cells transformed by a process in which said cell is brought into contact with a molecule selected from the group consisting of nucleic acids, polypeptides, glycosylated polypeptides, proteins and glycosylated proteins and a compound of formula:

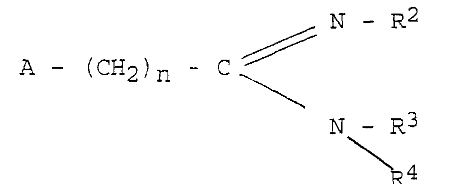

11. A process for the production of a vector in which a positively charged vesicle comprising at least one compound of formula: 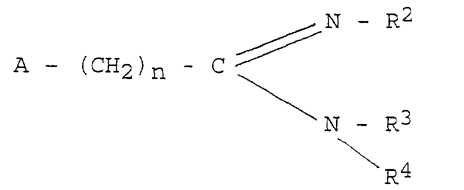 in which:
- A is selected from the group consisting of the radicals comprising a hydrophilic group or a CH₂ group and a hydrophobic group;
- n is a positive integer; and
- R², R³ and R⁴ are selected from the group consisting of a hydrogen atom, saturated alkyl chains, unsaturated alkyl chains and substituted alkyl chains,
is brought into contact with a molecule selected from the group consisting of nucleic acids, polypeptides, glycosylated polypeptides, proteins and glycosylated proteins.

## Patentansprüche

1. Vektor aus einem positiv geladenen Vesikel, das mindestens eine Verbindung nachfolgender Formel enthält: 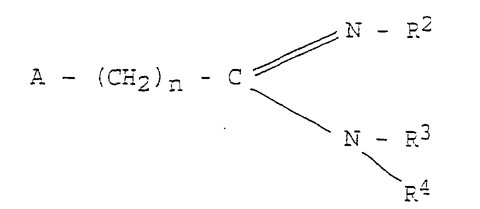 in welcher:
- A aus der Gruppe ausgewählt wird, bestehend aus den Radikalen mit einer hydrophilen Gruppe oder einer CH₂-Gruppe und einer hydrophoben Gruppe;
- n eine positive ganze Zahl ist;
- R², R³ und R⁴ aus der Gruppe ausgewählt werden, bestehend aus einem Wasserstoffatom, gesättigten Alkylketten, ungesättigten Alkylketten und substituierten Alkylketten,
wobei das genannte Vesikel mit mindestens einem Molekül kombiniert ist, das aus der Gruppe ausgewählt wird, bestehend aus Nukleinsäuren, Polypeptiden, glykosylierten Polypeptiden, Proteinen und glykosylierten Proteinen.

2. Vektor gemäß Anspruch 1, in welchem das Vesikel mindestens eine Verbindung nachfolgender Formel aufweist: 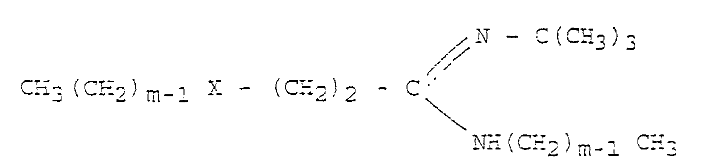 in welcher:
- m eine ganze Zahl zwischen 12 und 18 ist; und
- X aus der Gruppe ausgewählt wird, bestehend aus folgenden Verbindungen: 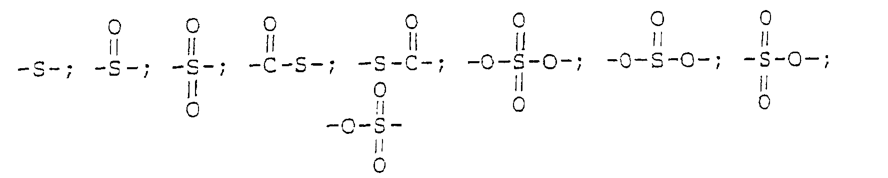 deren Selenanalogen, deren Telluranalogen,

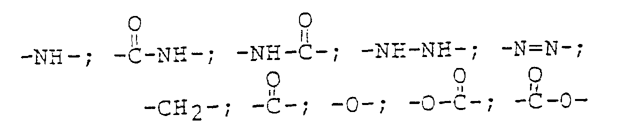

3. In-Vitro-Verfahren zur Einführung in eine Zelle, von mindestens einem Molekül, das aus der Gruppe ausgewählt wird, bestehend aus Nukleinsäuren, Polypeptiden, glykosylierten Polypeptiden, Proteinen und glykosylierten Proteinen, in welchem die genannte Zelle mit dem genannten Molekül und einer Verbindung folgender Formel in Kontakt gebracht wird: 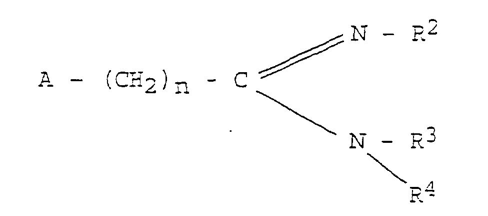 in welcher:
- A aus der Gruppe ausgewählt wird, bestehend aus den Radikalen mit einer hydrophilen Gruppe oder einer CH₂-Gruppe und einer hydrophoben Gruppe;
- n eine positive ganze Zahl ist;
- R², R³ und R⁴ aus der Gruppe ausgewählt werden, bestehend aus einem Wasserstoffatom, gesättigten Alkylketten, ungesättigten Alkylketten und substituierten Alkylketten.

4. Verfahren gemäß Anspruch 3, bei welchem die genannte Zelle mit dem genannten Molekül und einer Verbindung folgender Formel in Kontakt gebracht wird: 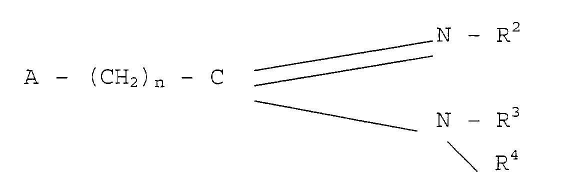 in welcher:
- A ein Radikal der Formel -X-R¹ darstellt, wobei R¹ aus der Gruppe ausgewählt wird, bestehend aus einem Wasserstoffatom, gesättigten Alkylketten, ungesättigten Alkylketten und substituierten Alkylketten, und wobei X aus folgender Gruppe ausgewählt wird: 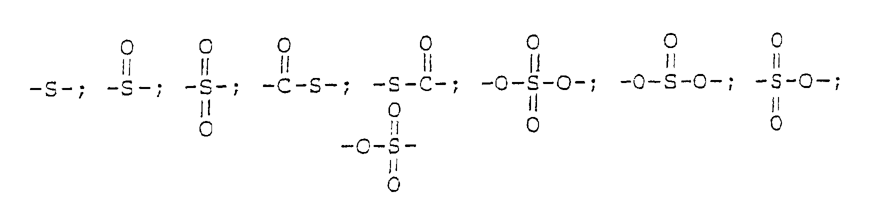 deren Selenanalogen, deren Telluranalogen,

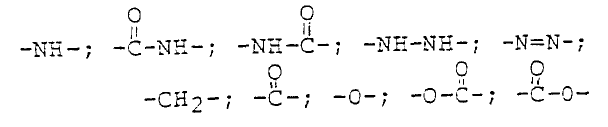

5. Verfahren gemäß Anspruch 3, bei welchem die genannte Zelle mit dem genannten Molekül und mit einem positiv geladenen Vesikel, in welchem die Membran mindestens eine Verbindung nachfolgender Formel aufweist, in Kontakt gebracht wird:

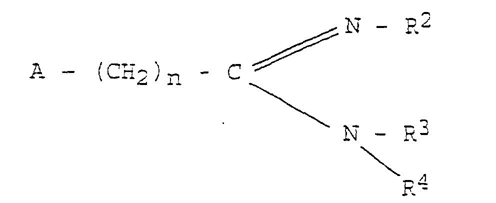

6. Verfahren gemäß Anspruch 5, bei welchem das Vesikel mindestens eine Verbindung nachfolgender Formel aufweist: 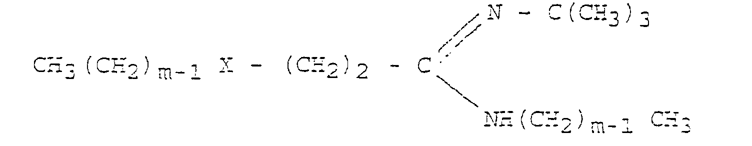 in welcher m eine ganze Zahl zwischen 12 und 18 ist.

7. Verfahren gemäß Anspruch 3, bei welchem die genannte Zelle mit einem Vektor, der aus einem positiv geladenen Vesikel besteht, bei welchem die Membran mindestens ein Molekül nachfolgender Formel aufweist, in Kontakt gebracht wird: 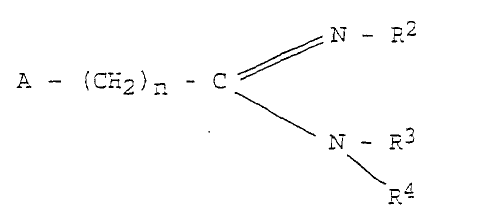 wobei das genannte Vesikel mit einem Molekül kombiniert wird, das aus der Gruppe ausgewählt wird, bestehend aus Nukleinsäuren, Polypeptiden, glykosylierten Polypeptiden, Proteinen und glykosylierten Proteinen.

8. Zelle, die durch ein Verfahren umgewandelt wird, wobei die genannte Zelle mit einem Molekül, das aus der Gruppe ausgewählt wird, bestehend aus Nukleinsäuren, Polypeptiden, glykosylierten Polypeptiden, Proteinen und glykosylierten Proteinen, und mit einer Verbindung folgender Formel in Kontakt gebracht wird: 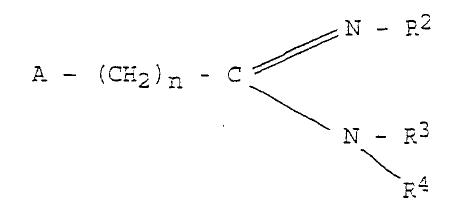 in welcher:
- A aus der Gruppe ausgewählt wird, bestehend aus den Radikalen mit einer hydrophilen Gruppe oder einer CH₂-Gruppe und einer hydrophoben Gruppe;
- n eine positive ganze Zahl ist;
- R², R³ und R⁴ aus der Gruppe ausgewählt werden, bestehend aus einem Wasserstoffatom, gesättigten Alkylketten, ungesättigten Alkylketten und substituierten Alkylketten.

9. Zelle gemäß Anspruch 8, wobei die genannte Zelle dadurch umgewandelt wird, dass sie mit einem Vektor in Kontakt gebracht wird, der aus einem positiv geladenen Vesikel besteht, bei welchem die Membran mindestens ein Molekül nachfolgender Formel enthält : 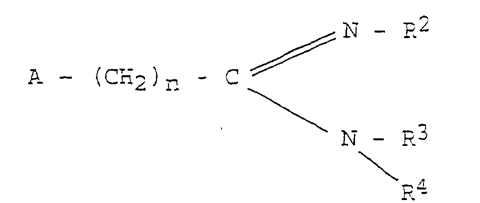 wobei das Vesikel mit einem Molekül kombiniert wird, das aus der Gruppe ausgewählt wird, bestehend aus Nukleinsäuren, Polypeptiden, glykosylierten Polypeptiden, Proteinen und glykosylierten Proteinen.

10. Pharmazeutische Zusammensetzung, die einen geeigneten pharmazeutischen Träger und ein Produkt enthält, die aus der Gruppe ausgewählt werden, die sich wie folgt zusammensetzt:
- aus den Vektoren, die aus einem positiv geladenen Vesikel bestehen und die mindestens eine Verbindung folgender Formel umfassen : 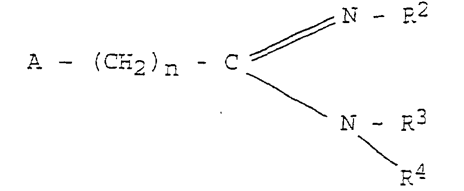
in welcher:
- A aus der Gruppe ausgewählt wird, bestehend aus den Radikalen mit einer hydrophilen Gruppe oder einer CH₂-Gruppe und einer hydrophoben Gruppe;
- n eine positive ganze Zahl ist;
- R², R³ und R⁴ aus der Gruppe ausgewählt werden, bestehend aus einem Wasserstoffatom, gesättigten Alkylketten, ungesättigten Alkylketten und substituierten Alkylketten,
wobei das genannte Vesikel mit mindestens einem Molekül der Nukleinsäure kombiniert ist, und
- den Zellen, die durch ein Verfahren umgewandelt werden,
wobei die genannte Zelle mit einem Molekül kombiniert wird, das aus der Gruppe ausgewählt wird, bestehend aus Nukleinsäuren, Polypeptiden, glykosylierten Polypeptiden, Proteinen und glykosylierten Proteinen, und mit einer Verbindung nachfolgender Formel in Kontakt gebracht werden:

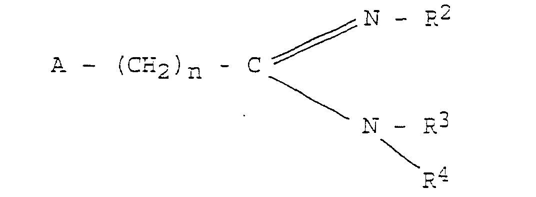

11. Verfahren für die Herstellung eines Vektors, bei welchem ein positiv geladenes Vesikel mindestens eine Verbindung nachfolgender Formel enthält : 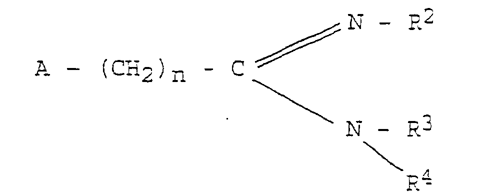 in welcher:
- A aus der Gruppe ausgewählt wird, bestehend aus den Radikalen mit einer hydrophilen Gruppe oder einer CH₂-Gruppe und einer hydrophoben Gruppe;
- n eine positive ganze Zahl ist;
- R², R³ und R⁴ aus der Gruppe ausgewählt werden, bestehend aus einem Wasserstoffatom, gesättigten Alkylketten, ungesättigten Alkylketten und substituierten Alkylketten,
mit einem Molekül in Kontakt gebracht wird, das aus der Gruppe ausgewählt wird, bestehend aus Nukleinsäuren, Polypeptiden, glykosylierten Polypeptiden, Proteinen und glykosylierten Proteinen.

## Revendications

1. Vecteur constitué d'une vésicule chargée positivement comprenant au moins un composé de formule : 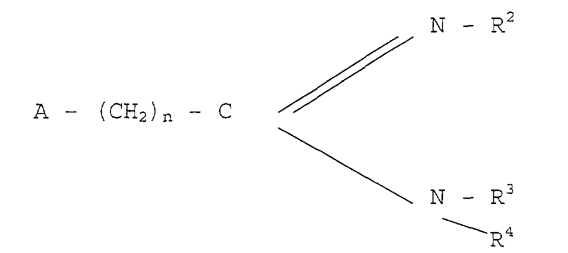 dans laquelle :
- A est choisi dans le groupe constitué des radicaux comprenant un groupe hydrophile ou un groupe CH₂ et un groupe hydrophobe;
- n est un nombre entier positif; et
- R², R³ et R⁴ sont choisis dans le groupe constitué d'un atome d'hydrogène, des chaînes alkyle saturées, des chaînes alkyle insaturées et des chaînes alkyle substituées,
ladite vésicule étant combinée avec au moins une molécule choisie dans le groupe constitué des acides nucléiques, des polypeptides, des polypeptides glycosylés, des protéines et des protéines glycosylées.

2. Vecteur selon la revendication 1, dans lequel la vésicule comprend au moins un composé de formule : 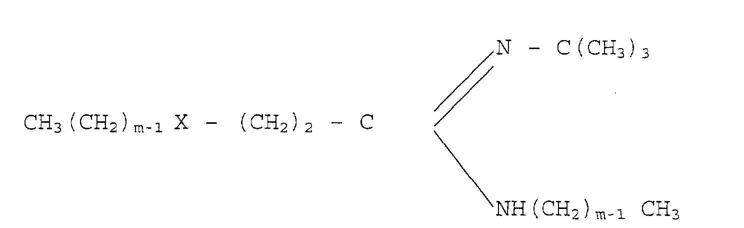 dans laquelle :
- m est un nombre entier de 12 à 18, et
- X est choisi dans le groupe constitué des radicaux suivants : 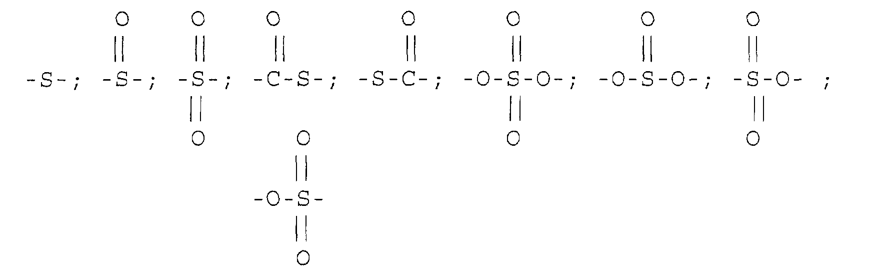 leurs analogues de sélénium, leurs analogues de tellure,

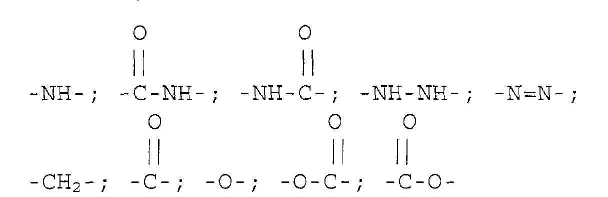

3. Procédé d'introduction d'au moins une molécule choisie dans le groupe constitué des acides nucléiques, des polypeptides, des polypeptides glycosylés, des protéines et des protéines glycosylées, in vitro, dans une cellule, dans lequel ladite cellule est mise en contact avec ladite molécule et un composé de formule : 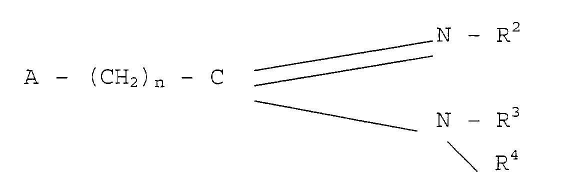 dans laquelle :
- A est choisi dans le groupe constitué des radicaux comprenant un groupe hydrophile ou un groupe CH₂ et un groupe hydrophobe;
- n est un nombre entier positif; et
- R², R³ et R⁴ sont choisis dans le groupe constitué d'un atome d'hydrogène, des chaînes alkyle saturées, des chaînes alkyle insaturées et des chaînes alkyle substituées.

4. Procédé selon la revendication 3, dans lequel ladite cellule est mise en contact avec ladite molécule et un composé de formule : 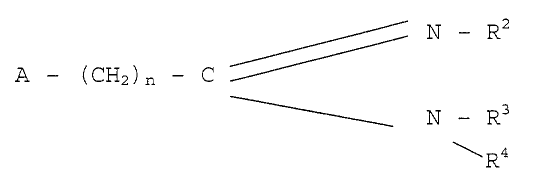 dans laquelle :
- A représente un radical de formule -X-R¹, R¹ étant choisi dans le groupe constitué d'un atome d'hydrogène, des chaînes alkyle saturées, des chaînes alkyle insaturées et des chaînes alkyle substituées, et X étant choisi dans le groupe constitué de: 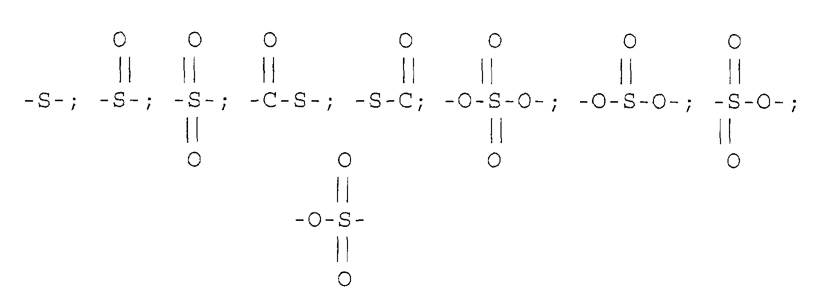 leurs analogues de sélénium, leurs analogues de tellure,

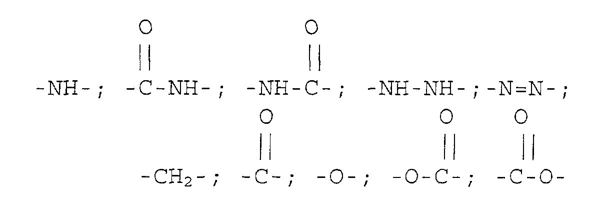

5. Procédé selon la revendication 3, dans lequel ladite cellule est mise en contact avec ladite molécule et avec une vésicule chargée positivement dont la membrane comprend au moins un composé de formule :

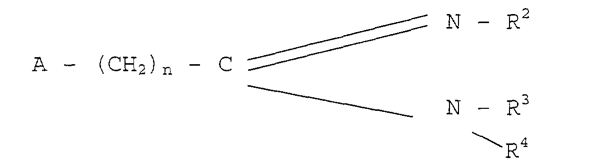

6. Procédé selon la revendication 5, dans lequel la vésicule comprend au moins un composé de formule : 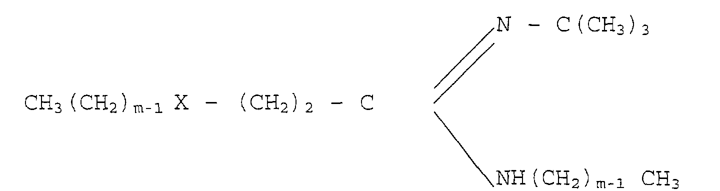 dans laquelle m est un nombre entier entre 12 à 18.

7. Procédé selon la revendication 3, dans lequel ladite cellule est mise en contact avec un vecteur constitué d'une vésicule chargée positivement dont la membrane comprend au moins une molécule de formule : 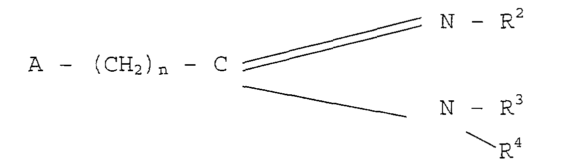 ladite vésicule étant combinée avec une molécule choisie dans le groupe constitué des acides nucléiques, des polypeptides, des polypeptides glycosylés, des protéines et des protéines glycosylées.

8. Cellule transformée par un procédé dans lequel ladite cellule est mise en contact avec une molécule choisie dans le groupe constitué des acides nucléiques, des polypeptides, des polypeptides glycosylés, des protéines et des protéines glycosylées et avec un composé de formule : 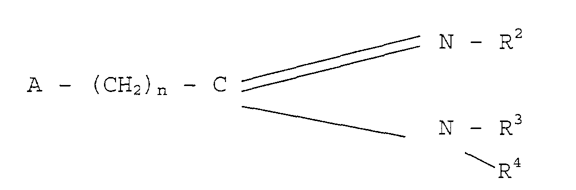 dans laquelle :
- A est choisi dans le groupe constitué des radicaux comprenant un groupe hydrophile ou un groupe CH₂ et un groupe hydrophobe;
- n est un nombre entier positif; et
- R2, R3 et R4 sont choisis dans le groupe constitué d'un atome d'hydrogène, des chaînes alkyle saturées, des chaînes alkyle insaturées et des chaînes alkyle substituées.

9. Cellule selon la revendication 8, ladite cellule étant transformée en la mettant en contact avec un vecteur constitué d'une vésicule chargée positivement dont la membrane comprend au moins une molécule de formule : 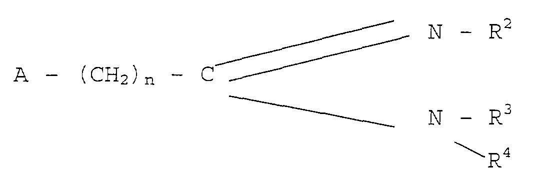 ladite vésicule étant combinée avec une molécule choisie dans le groupe constitué des acides nucléiques, des polypeptides, des polypeptides glycosylés, des protéines et des protéines glycosylées.

10. Composition pharmaceutique comprenant un véhicule pharmaceutique approprié et un produit choisi dans le groupe constitué :
- des vecteurs constitués d'une vésicule chargée positivement comprenant au moins un composé de formule : 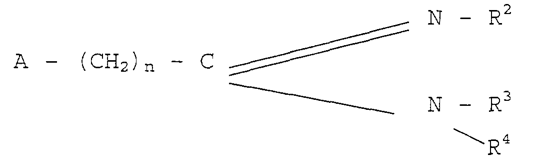
dans laquelle :
- A est choisi dans le groupe constitué des radicaux comprenant un groupe hydrophile ou un groupe CH₂ et un groupe hydrophobe;
- n est un nombre entier positif; et
- R², R³ et R⁴ sont choisis dans le groupe constitué d'un atome d'hydrogène, des chaînes alkyle saturées, des chaînes alkyle insaturées et des chaînes alkyle substituées,
ladite vésicule étant combinée avec au moins une molécule d'acide nucléique,
et
- les cellules transformées par un procédé dans lequel ladite cellule est mise en contact avec une molécule choisie dans le groupe constitué des acides nucléiques, des polypeptides, des polypeptides glycosylés, des protéines et des protéines glycosylées et avec un composé de formule :

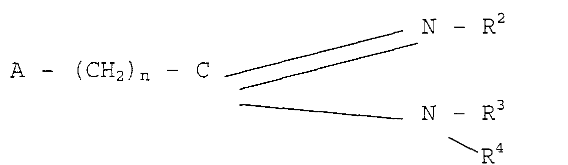

11. Procédé de production d'un vecteur dans lequel une vésicule chargée positivement comprenant au moins un composé de formule : 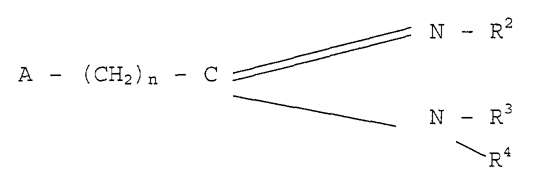 dans laquelle :
- A est choisi dans le groupe constitué des radicaux comprenant un groupe hydrophile ou un groupe CH₂ et un groupe hydrophobe;
- n est un nombre entier positif; et
- R², R³ et R⁴ sont choisis dans le groupe constitué d'un atome d'hydrogène, des chaînes alkyle saturées, des chaînes alkyle insaturées et des chaînes alkyle substituées,
est mise en contact avec une molécule choisie dans le groupe constitué des acides nucléiques, des polypeptides, des polypeptides glycosylés, des protéines et des protéines glycosylées.
